# EUROPEAN PATENT APPLICATION

(11) **EP 1 336 411 A1**
(43) Date of publication of application: **20.08.2003**
(21) Application number: 02075628.4
(22) Date of filing: 14.02.2002
(51) Int. Cl.: A61K 38/47, A61K 31/7032, A61P 43/00

(54) **Compositions and methods for improving enzyme replacement therapy of lysosomal storage diseases**

(71) Applicant: Academisch Medisch Centrum bij de Universiteit van Amsterdam, 1105 AZ Amsterdam (NL)
(72) Inventor: Aerts, Johannes, Maria, Franciscus, Gerardus, 1391 EJ Abcoude (NL)
(74) Representative: Prins, Adrianus Willem

(57) **Abstract**

The invention relates to the field of pharmaceutical products and therapy. The invention provides means and methods to increase the bio-availability of enzymes, more specifically lysosomal enzymes, more specifically alphaα-galactosidase-A. Pharmaceutical compositions are claimed that comprise a substance capable of preventing attachment of lysosomal enzyme to a binding molecule in the circulation. Use of these compositions with supplemented enzyme is also claimed.

## Description

The invention relates to the field of pharmaceutical products and therapy. In particular, the present invention relates to the field of enzyme supplementation therapy, more in particular, to the treatment of lysosomal enzyme deficiency disease, for example Fabry disease.

Protein supplementation therapy is a well known therapy for a variety of ailments. The best known example is probably the inoculation of insulin for the treatment of diabetes. The protein is normally inoculated in or near the circulation, for instance intramuscularly, and is taken up and distributed throughout the body by way of the blood circulation. For diabetes, the protein supplementation therapy is successful. Proteins with enzymatic activity have also been successfully employed in supplementation therapy. For example, type 1 Gaucher disease patients show a prominent clinical improvement following chronic administration of a human glucocerebrosidase enzyme preparation which compensates their inherited deficiency in this lysosomal hydrolase in pathological tissue macrophages. In many other cases, the therapy is less successful or fails altogether because the supplied enzyme is either instable in the circulation, or toxic, or its bio-availability is poor because the enzyme is bound to other substances. Bio-availability means that the enzyme is present in such chemical or physical form as to be capable of being utilized by a living organism.

Lysosomal enzymes are normally produced by all cells of the body. Lysosomal enzymes are synthesized at membrane-bound ribosomes and co-translationally translocated into the lumen of the endoplasmic reticulum. Via vesicular transport, the newly formed enzymes traverse the Golgi apparatus and undergo modifications in their N-linked glycans. In the Golgi apparatus the newly formed soluble lysosomal enzymes and non-enzymatic accessory proteins acquire a unique recognition signal, i.e. the formation of mannose-6-phosphate moieties in their high-mannose type N-linked glycans. A major proportion of the lysosomal enzymes is bound to mannose-6-phosphate specific receptors in the Golgi apparatus and is directly transported to late endosomes. Uncoupling of receptors and lysosomal enzyme ligand occurs and the latter is delivered to lysosomes whilst the receptor may recycle to Golgi apparatus. Part of the newly formed lysosomal enzymes is not directly delivered to lysosomes via the intracellular pathway but is secreted. The enzymes can be subsequently immediately endocytosed by mannose-6-phosphate receptors that are also expressed at the cell surface (Hasilik, 1980, Kaplan *et al*., 1977). Again, uncoupling from the receptor occurs in late endosomes and the receptor may recycle to the cell surface. This secretion-recapture pathway results in a constant level of lysosomal enzyme in the blood and other compartments of the body and makes the enzymes freely available for cellular uptake by other cells throughout the body (Holtzman, 1976). The acidity of the contents of a lysosome is lower (pH 5.0) than in other parts of the cell. The optimal activity level of lysosomal enzyme is reached at this pH 5.0 allowing the lysosomal enzymes to be optimally active in these organelles. The pH of circulating blood is around pH 7.2, far above the pH optimum of most lysosomal enzymes (Albert *et al*., 1983). The physiological importance of the mannose-6-phosphate receptor sorting of newly formed lysosomal enzymes is best illustrated by I-cell disease. In this inherited disorder the formation of mannose-6-phosphate moieties is impaired and consequently newly formed lysosomal enzymes are missorted. Enzyme levels in lysosomes are severely reduced whilst those in the circulation are dramatically elevated.

Some 60 different lysosomal enzymes are known today (Bainton, 1981), and they are all hydrolytic enzymes, including proteases, phospholipases, phosphatases, and sulfatases. Malfunctioning of any of these enzymes by inherited disorders causes various diseases, many of them accompanied by severe clinical manifestations. At present, over 40 inherited lysosomal storage disorders are known that are caused by defects in genes encoding a lysosomal hydrolase or some accessory protein required for optimal catalytic activity of a lysosomal hydrolase (Wraith, 2001). It has been observed that impaired lysosome functioning may also be caused by inherited defects in transporters of degradation products in the lysosomal membrane, defects in sorting mechanisms for newly formed lysosomal enzymes, defects in structural components of the lysosomal membrane or abnormalities in vesicular transport between lysosomes and endosomes (Wraith, 2001). Individuals suffering of an inherited lysosomal enzyme deficiency disease show severely reduced levels of functional enzyme in lysosomes. This may be caused by a variety of defects: absent or reduced transcription, reduced RNA stability or abnormal RNA splicing, production of truncated proteins, production of enzymes with a catalytic impairment, production of enzymes that are incorrectly sorted to lysosomes, production of enzymes with reduced intralysosomal stability or the formation of incorrectly folded enzymes that are predominantly prelysosomally degraded. The missing enzyme can be administered to cells of patients in vitro and these cells will take up the enzyme from the cell culture fluid and function normally with the added enzyme. Individuals with lysosomal enzyme disease may be treated by inoculating the missing enzyme intravenously (Neufeld *et al,.* 1975). This approach, named enzyme replacement therapy or enzyme supplementation therapy, has been successfully developed for the treatment of type 1 Gaucher disease that is characterized by a deficiency in the lysosomal hydrolase glucocerebrosidase (Barton, 1991). This success has stimulated research into application of enzyme replacement therapy for other lysosomal enzyme deficiencies. For example enzyme supplementation is developed for Fabry disease, an inherited disorder that is due to deficiency in alpha-Galactosidase A (GALA), a lysosomal enzyme that breaks down specific glycosphingolipids (Desnick *et al*., 1995, (Linthorst et al. 2000; Desnick, Ioannou, and Eng 1996).

Fabry disease.

The molecular basis of Fabry disease is a deficiency in the lysosomal hydrolase alpha-Galactosidase A. This hydrolase specifically degrades glycosphingolipids with a terminal, alpha-glycosidically linked galactose moiety. The membrane-component globotriaosylceramide (Gb3, or GL-3, or ceramidetrihexoside CTH) is the most important substrate. Other glycosphingolipids that are degraded by the enzyme are digalactosylceramide and blood group determinants B, B1 and P1.

As a result of the inherited enzyme deficiency, its glycosphingolipid substrates accumulate in lysosomes of several cell types of Fabry patients, but predominantly in endothelial cells. It is the endothelial cell lipid accumulation that is thought to be responsible for the most severe clinical manifestations of Fabry disease. The influence of blood group phenotype on the course of Fabry disease has not yet been described, though blood group B glycosphingolipids have been found to accumulate in patients with this blood group (Ledvinova et al. 1997).

The carrier frequency of Fabry disease is estimated to be 1:40.000 (Desnick, Ioannou, and Eng 1996). Due to the many ways the disease can present itself the disorder is probably under diagnosed. Several studies support this hypothesis. In two studies in Japan it is has been observed that alpha-Galactosidase A deficiency was the underlying cause for renal failure in 0,4% of a male dialysis population and for cardiac hypertrophy in 3% of males (Utsumi et al. 1999; Nakao et al. 1995). Preliminary reports confirm these results in European population.

The gene encoding for alpha-Galactosidase A is located on the X-chromosome, what makes males hemizygotes and females carriers of the disease. Currently in the Netherlands 30 hemizygotes and over 60 carriers have been identified (Linthorst et al. 2000). It is likely that these numbers rapidly increase because recent publications will lead to an increased physician awareness.

Fabry disease displays a broad clinical picture, both for hemizygotes and female carriers. In the older literature it is stated that female heterozygotes are often asymptomatic. However, recently this insight has completely changed. Female heterozygotes may indeed be asymptomatic, but most do experience symptoms of the disease, though often not as severe or later in life than hemizygotes (Linthorst et al. 2000; Desnick, Ioannou, and Eng 1996). The natural history of disease has been recently documented for hemizygotes and heterozygotes of Fabry disease (Mac Dermott, 2001a and 2001b).

In hemizygotes often the first presenting symptom is periodic excruciating pains in hand and feet (acroparesthesias) around the age of 10. These acroparesthesias can occur either spontaneously, but are often related to fever, heat or exercise. The pain is severe and is not relieved by standard medication. Only carbamazepine has shown to have a positive effect on relief of symptoms. Around puberty angiokeratoma can be found at the bathing trunk area and genitals of males. Angiokeratoma are teleangiectasies of the skin, caused by glycolipid depositions in the lysosomes of skin endothelial cells, which cause narrowing or occlusion of the lumen of the skin capillaries. The number of angiokeratomas increases with age. Other than being unaesthetic they pose no special threat. Fabry-patients show generally an absence of sweat-function (anhydrosis) and do not like to spend time in the sun or heat.

Later in life (around the age of 20-50) the more severe symptoms of Fabry disease occur due to the occlusion of small arteries in several organs. Progressive renal insufficiency occurs around the age of 40, sometimes earlier. In most males, sometimes also in females, this results in end-stage renal failure necessitating renal replacement therapy. Cardiac complications that generally develop in patients with Fabry disease include rhythm disturbances ranging from sinus bradycardia to a total AV block, which requires pacemaker implantation. Cardiac hypertrophy develops due to the accumulation of glycolipids in the myocytes, besides the endothelial cells. Patients with Fabry disease have an increased risk of myocardial infarction and death due to sudden rhythm abnormalities. Such cardiovascular complications may also occur in female heterozygotes. Lacunar (small) strokes have been shown to be present in almost all hemizygotes after the age of 30. In addition, white matter abnormalities can be found on MRI, which may be of vascular origin or a direct glycolipid-deposition in the brain cells (Tedeschi et al. 1999). Strokes may also occur with increased incidence in female heterozygotes.

The mean age of death in the Dutch male Fabry population (born after 1930) was 49 years (range 33 - 64). Main causes of death were rhythm disturbance or of other cardiac cause or uremia. There is a profound impact of Fabry disease on social life and of the only two out of the 11 known hemizygotes above the age of 40 are still able to work.

Until recently only symptomatic treatment, such as pain management or dialysis, was available. Renal transplantation does not have an effect on the deterioration of other organs. Apparently, the capacity of the transplanted kidney to produce alpha-Galactosidase A is not sufficient to reduce storage (and related morbidity) in other organs. Fundamental research on the alpha-Galactosidase A enzyme and gene (Bishop *et al*., 1986) has recently allowed to develop a rational strategy for therapeutic intervention by means of enzyme supplementation (as will be discussed below).

Laboratory diagnosis of Fabry disease is based on the demonstration of reduced levels of cellular alpha-Galactosidase A activity (Rietstra *et al.,* 1976). The disease can be also diagnosed by measuring enzyme levels in samples of urine (Desnick *et al*., 1971) or tears (Johnson *et al*., 1975). Prenatal enzymatic diagnosis is also feasible by direct analysis of chorionic villi (Kleijer *et al.* 1987). Research on the alpha-Galactosidase A gene has revealed that there are many 'private' mutations that are specific for a single pedigree. For example, only in the Netherlands 13 different mutations have been identified among Fabry families. Mutation analysis is particularly useful in identifying heterozygotes and prenatal testing.

Properties of alpha-Galactosidase A.

The features of the alpha-Galactosidase gene and enzyme have been described in great detail (see Desnick 1995). The gene encoding alpha-Galactosidase A is located on locus Xq22. The gene, encompassing 12 kb, contains seven exons. It contains several putative regulatory elements in the 5' flanking region, including a TAATAA sequence, five CCAAT box sequences and two GC box consensus sequences for the promoter-binding transcription factor Sp1. Several potential enhancer-binding sites are present, including the conserved recognition motif of the AP-1 enhancer-binding protein.

The full length cDNA encodes a 31- amino acid signal peptide and the 398 residues of the mature enzyme. The enzyme contains 3 N-linked glycans (N139, N192, N215) that are essential for the formation of a correct conformation that is compatible with enzymatic activity and stability. The enzyme molecules contain two high-mannose type glycans and one complex-type oligosaccharide chain. The molecular mass of the protein is about 51 kDa with SDS-PAGE. The native enzyme occurs as a homodimer with a total mass of about 110 kDa. Detailed information on the precise three-dimensional structure is still lacking since crystals have not yet been made that allow a high-resolution analysis by X-ray diffraction. Studies with mutant proteins created by site directed mutagenesis in the cDNA strongly indicate that D93 is the nucleophile involved in the hydrolytic mechanism. As expected for a lysosomal enzyme, alpha-Galactosidase A shows an acidic pH optimum for enzymatic activity. Maximal activity of alpha-Galactosidase A with the artificial substrate 4-methylumbelliferyl-alpha-D-galactopyranoside is obtained at pH 4.6 with an apparent Km of 2 mM. The apparent Km towards the natural lipid substrate is significantly lower. The in vitro hydrolysis of lipid substrate requires the addition of an appropriate detergent like sodium taurocholate. In vivo the cleavage is stimulated by the presence of a lysosomal activator protein such as SAP-1.

Studies on the life cycle of alpha-Galactosidase have indicated that its sorting to lysosomes is mediated by the formation of the mannose-6-phosphate recognition signal in its glycans. In cultured fibroblasts of patients with I-cell disease (a deficiency in mannose-6-phosphate formation) the majority of alpha-Galactosidase A is misrouted to the secretory pathway. The secreted enzyme (lacking mannose-6-phosphate) is not efficiently endocytosed after binding to mannose-6-phosphate specific cell surface receptors, thus causing elevated serum enzyme levels in these patients.

The alpha-Galactosidase A protein has been recombinantly produced in E.coli (Hantzopoulos and Calhoun, 1987), and using baculovirus vector (Coppola *et al*., 1994). Recombinant enzyme has also been produced after transfection of CHO (Chinese hamster ovary) cells. The precise composition of N-linked carbohydrates present on alpha-Galactosidase-A as produced in humans can not be formed upon recombinant production in bacteria or insect cell lines. Mannose-6-phosphate containing recombinant alpha-Galactosidase A can be generated upon expression of its cDNA in CHO cells.

Enzyme replacement therapy of Fabry disease is based on the concept of intravenous administration of recombinant alpha-Galactosidase A that should correct the intracellular deficiency in the enzyme. To ensure efficient uptake of enzyme by the many cell types that are directly involved in glycosphingolipid storage, the endocytosis of therapeutic enzyme via a general receptor-mediated uptake mechanism is favored. In this connection it has been envisioned that the mannose-6-phosphate receptor mediated uptake is ideally suited. This implies the presence of mannose-6-phosphate moieties in glycans of the recombinant enzyme. Such alpha-Galactosidase A can been produced by recombinant technology in Chinese hamster ovary (CHO) cells (Agalasidase beta; Fabrazyme, Genzyme corp.) as well as in cultured human skin fibroblasts with an activated promoter of the alpha-Galactosidase A gene (Agalasidase alpha, Replagal, Transkaryotic Therapies (TKT)). Recently both recombinant products have been registered in Europe by the EMEA and their registration in the USA by the FDA is pending. With both alpha-Galactosidases A clinical studies with Fabry patients have been conducted, based on repeated intravenous administration. Indeed, in both studies promising lipid substrate reductions in tissue biopsies have been observed (Eng et al. 2001; Schiffmann et al. 2001), (Pastores and Thadhani 2001). Due to the different dosage regimens and the different methods and clinical endpoints used in the two clinical trials, it is impossible to make a comparison between the two products on efficacy and safety. It is clear that both enzyme preparations reduce the amount of accumulated glycosphingolipid in affected organs in Fabry patients. However, evidence for clinical benefit is scarce and the duration of the clinical trials has been relatively short given the life-long nature of this form of treatment. Long-term results need to be awaited before a final judgment on the value of enzyme therapy can be made, let alone a difference between the two enzyme products.

The costs of treatment with enzyme therapy are huge for both commercial enzyme preparations; for example, the price for treating a 70 kg hemizygote Gaucher patient has been set at 160.000-200.000 $ a year. These enormous costs will surely affect the accessibility of therapy. In addition there are other concerns.
Side effects. Patients with Fabry disease usually lack any alpha-Galactosidase protein. Not surprisingly, most develop antibodies directed against the infused alpha-Galactosidase A preparations. This hypersensitivity leads to infusion related events, such as rigors and fever and may need additional treatment to overcome these reactions.
The current enzyme replacement treatment implies lengthy intravenous infusions once every two weeks. Either persons have to be trained to allow administration of the drug at home or the infusions have to be performed in the hospital. Even when a patient prefers home treatment, during the first 6 months the infusions will have to take place in the hospital because of potential complications related to hypersensitivity reactions. The therapy has therefore a profound impact on health resources and logistics as well as quality of life of patients.
Improvements in efficacy of therapy not only will have major economic effects but will also improve patients' quality of life. In addition, improved therapy will make initiation of treatment easier in the case of patients who are still not much affected by Fabry disease or in the case of female carriers.

In this patent application, we disclose that the alpha-Galactosidase A enzyme provided to patients with Fabry disease remains in circulating blood with a half-life time of 110 minutes (see also Schiffman *et al*., 1999, Eng *et al.,* 2001). The same enzyme, when infused intravenously in mice, has a half-life time of 10 minutes (Ioannou *et al.,* 2000). We disclose that human erythrocytes exhibit on their surface much more glycosphingolipids with terminal alpha-glycosidically linked galactose moieties, like ceramidetrihexoside (CTH), than mouse erythrocytes do. These glycosphingolipid substances are the normal substrate for alpha-Galactosidase A, so they are capable of binding to lysosomal enzyme and thus capable in extending its retention time in circulating blood. During this prolonged period in the circulating blood, the enzyme is not bioavailable for the cells in tissues, and is partly modified in N-linked glycan composition by catalytic enzymes. This catalytic digestion removes the sialic acid from complex type N-linked glycans present on alpha-Galactosidase A, favoring subsequent uptake via the asialoglycoprotein receptor on liver hepatocytes. Selective uptake of therapeutic enzyme by hepatocytes is not beneficial for the patients since these cells do not store excessively glycosphingolipid in Fabry patients and do not contribute to the pathology. Another modification that may occur during retention of alpha-Galactosidase by binding to erythrocytes is the removal of phosphate groups from the high-mannose type N-linked carbohydrate chains present on the enzyme. This diminishes the efficiency of the therapeutic intervention that is based on selective uptake of the enzyme from the bloodstream by binding to mannose-6-phosphate receptors that are ubiquitously expressed by cell types, including those directly involved in the pathology such as endothelial cells. The loss of mannose-6-phosphate moieties from intravenously administered alpha- Galactosidase preparations should be considered to be very disadvantageous for the efficacy of intervention. Not only will it reduce the selective uptake of enzyme by the desired cell types, but even if the enzyme would reach the cells and would be taken up, the enzyme would not be efficiently directed to the lysosomes, because this process is also directed by a mannose-6-phosphate receptor (Albert *et al*., 1983). Cells like macrophages have a mannose receptor, that will recognize mannose without the phosphate group, and they will preferentially take up the modified enzyme and destroy it. Both preferential uptake by hepatocytes (asialoglycoprotein receptor-mediated) and macrophages (mannose-receptor mediated) renders the enzyme supplementation treatment for Fabry patients less effective.

The invention provides a pharmaceutical composition comprising a binding molecule capable of competing with a blood component for a binding site of an enzyme, said enzyme having its desired site of action outside the blood circulation, said enzyme being capable of binding a substrate at its site of action, said enzyme having a lower affinity for said binding molecule than for its substrate at its site of action.

The invention further provides a pharmaceutical composition comprising said binding molecule, and further comprising said enzyme.

More specifically, the invention provides said pharmaceutical composition wherein said enzyme comprises a lysosomal enzyme, more specifically alpha-Galactosidase-A.

More specifically, the invention provides said pharmaceutical composition wherein said blood component comprises ceramidetrihexoside (CTH). To prevent the enzyme from binding to CTH, which is present on the erythrocytes, a CTH-homologue is used that is not capable of binding to erythrocytes.

The invention provides said pharmaceutical composition wherein said binding molecule comprises a substrate homologue like for example CTH-sphingosine and/or CTH-sphingosine-X or a functional part, derivative and/or analogue thereof. The desired site of action of said enzyme in said pharmaceutical composition comprises the cell, more specifically a cellular component, more specifically a lysosome.

Because said binding molecule is capable of blocking the active site of the enzyme, it is also capable of decreasing the activity of the enzyme during its stay in the circulation, thereby decreasing deleterious side-effects of the enzyme.

In another embodiment the invention provides identification of suitable blockers of the catalytic site of a lysosomal enzyme.

For any given lysosomal enzyme a suitable blocker of the catalytic site can be identified by screening compounds for their ability to competitively inhibit enzymatic activity of the lysosomal enzyme. Competitive inhibition can be established by analysis of Michaelis-Menten kinetics (see for example Darnell, J., Lodish, H. and Baltimore, D. Molecular Cell Biology, Scientific American Books, New York (1986). Compounds should show a high inhibitory capacity at (near) neutral pH. The blocking effect of compounds can be further validated by analysis of the effect on clearance from the circulation of intravenously administered enzyme with and without prior saturation with the compound.

The invention also provides for a pharmaceutical composition comprising a binding molecule capable of decreasing or preventing any deleterious effects caused by enzyme after parenteral application.

In another embodiment, the invention provides for the use of said pharmaceutical composition for the preparation of a medicament, said medicament being used for the treatment of lysosomal deficiency disorders, more specifically for the treatment of Fabry disease.

In yet another embodiment, the invention provides for a method to increase the bio-availability of enzymes, more specifically lysosomal enzymes, more specifically alpha-Galactosidase A, comprising providing said medicament to individuals by parenteral administration, said individuals suffering of a disease, more specifically suffering of a lysosomal disorder, more specifically of Fabry disease.

The invention is further explained in the detailed description herein without limiting the invention.

The concept of improved protein replacement therapy by increasing bio-availability of an enzyme following saturation of its catalytic center prior to intravenous administration is applicable to all inherited storage disorders that are caused by a deficiency in a lysosomal enzyme. Examples are MPS I (Hurler, Scheie, Hurler/Scheie; iduronidase deficiency), MPS II (Hunter; iduronate-2-sulphatase deficiency), MPS III (Sanfillipo types A, B, C and D, respectively; heparan-n-sulphatase; N-acetylglucosaminidase; AcetylCoA glucosamine n-acetyl transferase; N-acetylglucosamine-6-sulphatase deficiencies, respectively), MPS IV (Morquio types A and B, respectively; galactose-6-sulphatase, beta-galactosidase deficiencies, respectively), MPS VI (Maroteaux-Lamy; galactosamine-4-sulphatase deficiency), MPS VII (Sly; beta-glucuronidase deficiency), MPS IX (hyaluronidase deficiency), Mucolipidosis I (Sialidosis I; neuraminidase deficiency), ML II (I-cell; transferase deficiency), ML III (pseudo-Hurler transferase deficiency), GM1-gangliosidosis (beta-galactosidase deficiency), GM2-ganagliosidosis (Tay-Sachs; beta-hexosaminidase A deficiency), (Sandhoff; beta-hexosaminidase A and B deficiency) Krabbe (galactocerebrosidase deficiency) MLD (arylsulphatase deficiency), Fabry (alpha-galactosidase A deficiency), Gaucher (glucocerebrosidase deficiency), Farber (ceramidase deficiency), Niemann Pick types A and B (acid sphingomyelinase deficiency) alpha-Mannoidosis (alpha-mannosidase deficiency), beta-Mannosidosis (beta-mannosidase deficiency), Fucosidosis (fucosidase deficiency), Aspartylglucosaminuria (aspartylglucosaminidase deficiency), Schindler disease (alpha-Galactosidase B deficiency), Pompe disease (acid alpha-glucosidase deficiency), Wolman disease and cholesterol ester storage disease (acid lipase deficiency), Pycnodysostosis (Cathepsin deficiency), CLN1 infantile form; palmitoyl protein thioesterase deficiency), CLN2 (late infantile form; pepstatin insensitive carboxypeptidase deficiency), Multiple sulphatase deficiency (multiple sulphatase enzyme deficiencies).

The innovative invention implies that prior to the intravenous administration of the therapeutic enzyme its catalytic site is saturated with a substrate analogue that will help to prevent deleterious interaction with chemical structures in the bloodstream that result in undesired retention and reduced targeting to lysosomes. The saturation may moreover help to stabilize the enzyme in its active conformation in the circulation and moreover may prevent non-physiological enzyme catalyzed reactions in the circulation.

Besides the example described in detail for Fabry disease, other examples are the saturation of acid sphingomyelinase with a sphingomyelin analogue to improve the outcome of enzyme therapy of Niemann Pick disease types A and B. The invention will reduce the undesired retention in the circulation and thus promote the beneficial uptake and delivery to lysosomes. Inside the lysosome the sphingomyelin analogue will be hydrolyzed and the enzyme will be unblocked and able to hydrolyze the stored sphinogomyeline. Another example is Pompe disease, undesired interactions of acid alpha-glucosidase via its catalytic center with glycan structures in the circulation reduce the therapeutic efficacy of enzyme replacement therapy. Blocking of this interaction with a substrate analogue will decrease the harmful interaction and optimalize the outcome of enzyme replacement therapy. Optimal therapeutic blockers of recombinant enzyme in this case would be compounds containing an alpha-glucoside that firmly interact at neutral pH with the catalytic site and are released in the acidic lysosome, for example after hydrolytic release of the alpha-glucoside moiety.

### Detailed description

### MATERIALS & METHODS

### Purification of human alpha-Galactosidase A

Native human alpha-Galactosidase A was isolated from 7,000 ml urine from a healthy male. Urine was concentrated using the Amicon Diaflo system (Danvers, MA, USA) with a 10 kDa cutoff membrane and washed with a 100 mM potassium-phosphate buffer, pH 6.5, containing 50 mM NaCl (= Buffer A). The concentrated urine was then loaded onto a 30 ml ConA-Sepharose column which had been equilibrated with Buffer A. Unbound proteins were removed by washing with 10 column volumes of Buffer B (100 mM potassium phosphate pH 6.5; 500 mM NaCl). Bound proteins were eluted from the ConA column with Buffer B containing 100 mM methylmannoside and collected in 1 ml fractions. Fractions containing alpha-Galactosidase A activity were pooled and concentrated using Millipore Ultrafree-MC centrifugal filter units (Bedford, MA, USA) with a molecular cutoff of 10 kDa. This concentrated material was then subjected to Iso Electric Focussing, using Servalyt 4-9 T (Serva, Heidelberg, Germany) as carrier ampholytes. After focussing, the gel was divided into 30 fractions and proteins were extracted by the addition of 1 ml water followed by centrifugation. The supernatants were then tested for alpha-Galactosidase A activity. Positive fractions were pooled, TCA precipitated and further separated on a 10% SDS-PolyAcrylAmide gel. After silverstaining, the alpha-Galactosidase A protein band (as identified by Western blotting of parallel lanes) was excised and subjected to mass spectrometry (see below).

### Production of recombinant alpha-Galactosidase A in COS-1 cells

Transient transfection of COS-1 cells with alpha-Galactosidase A constructs was performed by the DEAE-Dextran method, essentially as described in Lopata *et al*, 1984. Enzyme was isolated from the medium using the procedure described above (ultrafiltration, Concanavalin A Sepharose chromatograpy and preparative isoelectric focussing).

### Production of human hexosaminidase.

Spleen (10 gr) from a type 1 Gaucher patient was homogenized by ultrasonication in 100 ml phosphate buffered saline. The homogenate was centrifuged for 15 minutes at 10.000 g and the supernatant was collected. The material was subjected to Concanavalin A Sepharose chromatography as described above.

### Enzyme activity measurements.

The enzymatic activity of alpha-Galactosidase A secreted into the medium was measured using the fluorogenic substrate 4-MethylUmbelliferyl-β-D-galactopyranoside (4-MU-galactopyranoside; Sigma, St. Louis, MO, USA), essentially as described (Ioannou *et al*., 1992). Briefly, 25 µl of a test fraction was incubated with 100 µl reaction mixture containing the 4-MU substrate (final concentration 3.5 mM) in 100 mM Citrate/200 mM Phosphate buffer pH 4.6 at 37 °C for 1 hour. Reactions contained 100 mM N-acetylgalactosamine to inhibit alpha-Galactosidase B activity (Mayes et al., 1981). Reactions were terminated by the addition of 2 ml 300 mM Glycine/NaOH buffer, pH 10.6 and fluorescent 4-methyl-umbelliferone was measured with a fluorimeter (Perkin-Elmer Corp., Norwalk, CT, USA) at 445 nm.

Enzymatic activity of hexosaminidase was measured using the fluorgenic substrate 4-MethylUmbelliferyl-beta-hexosaminide; Sigma, St.Louis, MO, USA). Briefly, 25 µl of a test fraction was incubated with 100 µl reaction mixture containing the 4-MU substrate (final concentration 3.5 mM) in 100 mM Citrate/200 mM Phosphate buffer pH 4.0 at 37 °C for 1 hour. Reactions were terminated by the addition of 2 ml 300 mM Glycine/NaOH buffer, pH 10.6 and fluorescent 4-methyl-umbelliferone was measured with a fluorimeter (Perkin-Elmer Corp., Norwalk, CT, USA) at 445 nm.

### Erythrocyte isolation.

Erythrocytes were prepared from freshly drawn blood of volunteers to which EDTA was added. The sample was centrifuged at 3000 rpm for 10 minutes and the (plasma) supernatant and buffy coat (leukocytes, thrombocytes) were carefully removed. Erythrocytes were resuspended in phosphate buffered saline and the centrifugation was repeated. After removal of the supernatant the cells were resuspended in an
identical volume phosphate buffered saline and stored at 4 C.

### Affinity chromatographic assay for the interaction of alpha-Galactosidase A with erythrocytes

An affinity column is prepared by firstly layering Sepharose beads in a glass column. Next 5 ml's of a 1:1 (v/v) suspension of human erythrocytes in phosphate buffered saline was applied. The erythrocytes are allowed to sediment and the column was gently washed with 10 ml of phosphate buffered saline. A mixture is prepared of purified native urinary alpha-Galactosidase A, or purified recombinant alpha-Galactosidase A, together with purified hexosaminidase is prepared in phosphate buffered saline that contains an identical amount of enzymatic activity : 10 nmol substrate hydrolysis per hour by hexosaminidase and by alpha-Galactosidase A. The enzyme mixture also contained 10 % (w/v) sucrose to increase the buoyancy. The enzyme mixture (1 ml) is applied to the column at a flow rate of 0.2 ml per minute. Next was applied phosphate buffered saline containing as dye cytochrome c. Fractions of o.1 ml were collected and analyzed on enzymatic activity using the assay conditions described above. Affinity interaction is detected by a slower elution of alpha-Galactosidase A from the erythrocyte column as compared to hexosaminidase.

### Detection of competitor of alpha-Galactosidase A retention on erythrocytes.

The affinity chromatographic procedure described above is employed. To the alpha-Galactosidase A-hexosaminidase mixture are added compounds that interact with the catalytic center and thus may lower the affinity interaction of alpha-Galactosidase A with erythrocytes. A likely candidate is the potent competitive inhibitor CTH-sphingosine that should saturate the catalytic center of alpha-Galactosidase A at 200 micromolar concentration.

**References**
1. Albert B, Bray D, Lewis J, Raff M, Roberts K, Watson JD (Eds). Molecular biology of the cell. Farland Publishing Inc. New York & London (1983) pp 3767-372.Bainton D (1981): The discovery of lysosomes. J. Cell. Biol. 91: 66s-76s.
2. Barton NW, Brady RO, Dambrosia JM, Di Bisceglie AM, Doppelt SH, Hill SC, Mankin HJ, Murray GJ, Parker RI, Argoff CE, et al. (1991): Replacement therapy for inherited enzyme deficiency--macrophage-targeted glucocerebrosidase for Gaucher's disease. N Engl J Med 324 (21):1464-70
3. Bishop DF Bishop DF, Calhoun DH, Bernstein HS, Hantzopoulos P, Quinn M, Desnick RJ.et al (1986): Human alpha-galactosidase A: nucleotide sequence of a cDNA clone encoding the mature enzyme. Proc. Natl. Acad. Sci. USA 83 (13): 4859-4863.
4. Coppola G, Yan Y, Hantzopoulos P, Segura E, Stroh JG, and Calhoun DH (1994): Characterization of glycosylated and catalytically active recombinant human α-galactosidase-A using a baculovirus vector. Gene 144: 197-203.
5. Desnick RJ, Ioannou YA, Eng CM (1995): α-galactosidase-A deficiency: Fabry disease. In Scriver CR, Boudet AL, Sly WS, and Valle D (Eds.) The metabolic basis of inherited disease, II, seventh edition, McGraw-Hill, New York pp.2741-2784.
6. Desnick RJ, Ioannou YA, and Eng ME. (1996): alpha-Galactosidase A Deficiency: Fabry disease. in: Scriver CR, Beaudet AL, Sly WS, Valle D, eds.The metabolic and molecular bases of inherited disease. 6th ed.New York: McGraw-Hill: 2741-2784.
7. Desnick RJ, Dawson G, Desnick SJ, Sweeley CC, Krivit W (1971): Diagnosis of glycosphingolipidoses by urinary sediment analysis. N. Engl. J. Med. 284: 739.
8. Eng CM, Guffon N, Wilcox WR, Germain DP, Lee P, Waldek S, Caplan L, Linthorst GE, Desnick RJ; International Collaborative Fabry Disease Study Group. (2001): Safety and efficacy of recombinant human alpha-galactosidase A-- replacement therapy in Fabry's disease. N. Engl. J. Med. 345 (1): 9-16.
9. Hantzopoulos PA, and Calhoun DH (1987): Expression of the human α -galactosidase-A in Escherichia coli K-12. Gene 57: 159-169.
10. Hasilik A (1980): Biosynthesis of lysosomal enzymes. Trends in Biochem. Sci. 5: 237-240.
11. Ho MW (1973): Hydrolysis of ceramide trihexoside by a specific a-galactosidase from human liver. Biochem. J. 133: 1.
12. Holtzman E. (1976): Lysosomes: A survey. New York: Springer-Verlag.
13. Ioannou YA, Zeidner KM, Gordon RE, Desnick RJ. (2001): Fabry disease: preclinical studies demonstrate the effectiveness of alpha-galactosidase A replacement in enzyme-deficient mice. Am. J. Hum. Genet. 68 (1): 14-25.
14. Johnson DL, Del Monte MA, Cotlier E, Desnick RJ (1975): Fabry disease: Diagnosis of hemizygotes and heterozygotes by a-galactosidase A activity in tears. Clin. Chim. Acta 63: 81.
15. Kaplan A, Achord DT, Sly WS (1977): Phosphohexosyl components of a lysosomal enzyme are recognized by pinocytosis receptors on human fibroblasts. Proc. Natl. Acad. Sci. USA 74: 2026-2030.
16. Kleijer WJ, Hussaarts-Odijk LM, Sacks ES, Jahoda MGJ, Niermeijer MF (1987): Prenatal diagnosis of Fabry's disease by direct analysis of chorionic villi. Prenat. Diag. 7: 283.
17. Ledvinova J, Poupetova H, Hanackova A, Pisacka M, Elleder M. (1997): Blood group B glycosphingolipids in alpha-galactosidase deficiency (Fabry disease): influence of secretor status. Biochim. Biophys. Acta 1345 (2):180-187.
18. Linthorst GE, Hollak CE, Bosman DK, Heymans HS, Aerts JM. (2000): De ziekte van Fabry: op weg naar een behandeling. Ned. Tijdschr. Geneeskd. 144 (50): 2391-2395.
19. Lopata MA, Cleveland DW, Sollner-Webb B. (1984): High level transient expression of a chloramphenicol acetyl transferase gene by DEAE-dextran mediated DNA transfection coupled with a dimethyl sulfoxide or glycerol shock treatment. Nucleic Acids Res. 12 (14): 5707-17.
20. MacDermot KD, Holmes A, Miners AH. (2001a): Anderson-Fabry disease: clinical manifestations and impact of disease in a cohort of 98 hemizygous males. J Med Genet 38(11):750-60
21. MacDermot KD, Holmes A, Miners AH. (2001b): Anderson-Fabry disease: clinical manifestations and impact of disease in a cohort of 60 obligate carrier females. J Med Genet 38(11):769-75
22. Mayes JS, Scheerer JB, Sifers RN, Donaldson ML. (1981): Differential assay for lysosomal alpha-galactosidases in human tissues and its application to Fabry's disease. Clin. Chim. Acta 5; 112 (2): 247-51.
23. Nakao S, Takenaka T, Maeda M, Kodama C, Tanaka A, Tahara M, Yoshida A, Kuriyama M, Hayashibe H, Sakuraba H, et al. (1995): An atypical variant of Fabry's disease in men with left ventricular hypertrophy. N. Engl. J. Med. 333 (5): 288-293.
24. Neufeld EF, Lim TW, Shapiro TJ (1975): Inherited disorders of lysosomal metabolism. Annu. Rev. Biochem. 44: 357-376.
25. Pastores GM and Thadhani R (2001): Enzyme-replacement therapy for Anderson-Fabry disease. The Lancet 358 (9282): 601-603.
26. Rietstra PJGM, Brouwer-Kelder EM, de Groot WP, Tager JM (1976): The use of biochemical parameters for the detection of carriers of Fabry's disease. J. Mol. Med. 1:237.
27. Schiffmann R, Kopp JB, Austin HA 3rd, Sabnis S, Moore DF, Weibel T, Balow JE, Brady RO. (2001): Enzyme replacement therapy in fabry disease: a randomized controlled trial. JAMA 285 (21): 2743-2749.
28. Tedeschi G, Bonavita S, Banerjee TK, Virta A, Schiffmann R. (1999): Diffuse central neuronal involvement in Fabry disease: a proton MRS imaging study. Neurology 52 (8): 1663-1667.
29. Utsumi K, Kase R, Takata T, Sakuraba H, Matsui N, Saito H, Nakamura T, Kawabe M, Iino Y, Katayama Y. (1999): Fabry disease in patients receiving maintenance dialysis. Clinical and Experimental Nephrology 4 (1):49-51.
30. Wraith JE. (2001): Advances in the treatment of lysosomal storage disease. Dev Med Child Neurol. 43(9):639-946.

## Claims

1. A pharmaceutical composition comprising a binding molecule capable of competing with a blood component for a binding site of an enzyme, said enzyme having its desired site of action outside the blood circulation.

2. A pharmaceutical composition according to claim 1 wherein said enzyme is capable of binding a substrate at its site of action outside the blood circulation.

3. A pharmaceutical composition according to claim 1 or 2 wherein, at its site of action outside the blood circulation, said enzyme has a lower affinity for said binding molecule than for its substrate.

4. A pharmaceutical composition according to anyone of claim 1-3 further comprising said enzyme.

5. A pharmaceutical composition according to anyone of claim 1-4 wherein said enzyme comprises a lysosomal enzyme.

6. A pharmaceutical composition according to claim 5 wherein said enzyme comprises α-galactosidase-A.

7. A pharmaceutical composition according to anyone of claim 1-6 wherein said blood component comprises CTH.

8. A pharmaceutical composition according to anyone of claim 1-7 wherein said binding molecule comprises CTH-sphingosine and/or CTH-sphingosine-X or a functional part thereof.

9. A pharmaceutical composition according to anyone of claim 1-8 wherein said site of action outside the blood circulation comprises a cell.

10. A pharmaceutical composition according to anyone of claim 1-9 wherein said site of action outside the blood circulation comprises a cellular compartment.

11. A pharmaceutical composition according to anyone of claim 1-10 wherein said site of action outside the blood circulation comprises a lysosome.

12. A pharmaceutical composition according to anyone of claim 1-11 wherein said binding component is capable of decreasing a deleterious effect of said enzyme after parenteral application.

13. Use of a pharmaceutical composition according to anyone of claim 1-12 for the preparation of a medicament.

14. Use of a pharmaceutical composition according to claim 13 for the preparation of a medicament for the treatment of a lysosome enzyme deficiency disease.

15. Use of a pharmaceutical composition according to claim 14 wherein said lysosome enzyme deficiency disease comprises Fabry disease.

16. A method to increase bio-availability of enzyme, comprising providing a pharmaceutical composition according to anyone of claim 1-12 by parenteral administration.

17. A method according to claim 16, wherein said enzyme comprises lysosomal enzymes.

18. A method according to claim 16 or 17, wherein said enzyme comprises α -galactosidase-A

19. A method according to anyone of claims 16-18, for the treatment of disease.

20. A method according to anyone of claims 16-18, for the treatment of lysosomal deficiency disease.

21. A method according to anyone of claim 16-18, for the treatment of Fabry disease.
